# EUROPEAN PATENT APPLICATION

(11) **EP 4 092 176 A1**
(43) Date of publication of application: **23.11.2022**
(21) Application number: 22173648.1
(22) Date of filing: 16.05.2022
(51) Int. Cl.: D06F 33/65, D06F 58/20, D06F 35/00, D06F 103/02, D06F 103/64, D06F 103/66

(54) **LAUNDRY TREATING APPLIANCE AND METHOD**

(30) Priority: 18.05.2021 US 202117323571
(71) Applicant: WHIRLPOOL CORPORATION, Benton Harbor Michigan 49022 (US)
(72) Inventor: Giacomini, Neomar, 21024 Cassinetta di Biandronno (VA) (IT); Vehslage, David, 21024 Cassinetta di Biandronno (VA) (IT)
(74) Representative: Spina, Alessandro

(57) **Abstract**

A laundry treating appliance (10) and method of initiating a sanitization cycle in a laundry treating appliance (10) includes sensing at least one of a presence of an article within a sanitizing compartment, an identification of the article within the sanitizing compartment, or a chemical property within the sanitizing compartment, and determining, in a controller (106), a need for initiating a sanitization cycle.

## Description

### BACKGROUND

Laundry treating appliances, such as washing machines, combination washer/dryers, refreshers, and non-aqueous systems, can have a configuration based on a rotating drum that at least partially defines a treating chamber in which laundry items or other articles are placed for treating. The laundry treating appliance can have a controller that implements a number of user-selectable, pre-programmed cycles of operation having one or more operating parameters. Hot water, cold water, or a mixture thereof, along with various treating chemistries, can be supplied to the treating chamber in accordance with the cycle of operation. In addition, hot air, cold air, or a mixture thereof can be supplied to the treating chamber in accordance with the cycle of operation and via an air flow assembly.

### BRIEF SUMMARY

In one aspect, the present disclosure relates to a method of initiating a sanitization cycle in a laundry treating appliance having a sanitizing compartment. The method includes automatically sensing, via a sensor, at least one of a presence of an article within the sanitizing compartment, an identification of the article within the sanitizing compartment, or a chemical property within the sanitizing compartment, receiving, in a controller, a signal from the sensor indicating the at least one of the presence of the article, the identification of the article, or the chemical property, determining, in the controller, a need for initiating a sanitization cycle within the sanitizing compartment based on the signal from the sensor, and controllably operating, by the controller, an ionizing light source within the laundry treating appliance based on the determined need for initiating the sanitization cycle.

### BRIEF DESCRIPTION OF THE DRAWINGS

In the drawings:
FIG. 1 illustrates a schematic cross-sectional view of a laundry treating appliance in the form of a combination washing and drying machine in accordance with various aspects described herein.
FIG. 2 illustrates a schematic of a control system of the laundry treating appliance of FIG. 1.
FIG. 3 is a perspective view of the laundry treating appliance of FIG. 1 with a sanitization system in accordance with various aspects described herein.
FIG. 4 is a perspective view of the laundry treating appliance of FIG. 1 with another sanitization system in accordance with various aspects described herein.
FIG. 5 is a schematic diagram illustrating the sanitization system of FIG. 4.

### DETAILED DESCRIPTION

Aspects of the disclosure relate to a laundry treating appliance with a sanitization system and a method of initiating a sanitization cycle therein. For the purposes of illustration, aspects of the disclosure will be described in the exemplary environment of a combination washing and drying machine. It will be understood that the disclosure is not so limited, and that aspects of the disclosure can be utilized in any appliance which performs a cycle of operation to clean or otherwise treat items placed therein, including a stand-alone washer, a horizontal-axis or vertical-axis washer; a combination washing machine and dryer; a stand-alone dryer, a tumbling or stationary refreshing/revitalizing machine; an extractor; a non-aqueous washing apparatus, or a revitalizing machine, in non-limiting examples.

Laundry treating appliances can be provided with structures and functionality both for washing and drying laundry items within a single appliance. In the case of such a combination washing and drying appliance, in addition to the components provided in a traditional washing machine, additional components for drying laundry items are also provided within the appliance. Non-limiting examples of such drying components include an air flow pathway, including an air inlet and an air outlet to the tub interior, a condenser, a blower, a heating element, and a manifold.

Washing machines are typically categorized as either a vertical axis washing machine or a horizontal axis washing machine. The terms vertical axis and horizontal axis are often used as shorthand terms for the manner in which the appliance imparts mechanical energy to the load of laundry, even when the relevant rotational axis is not absolutely vertical or horizontal. As used herein, a "vertical axis" washing machine refers to a washing machine having a rotatable drum, perforate or imperforate, that holds fabric items and a clothes mover, such as an agitator, impeller, nutator, and the like within the drum. The clothes mover moves within the drum to impart mechanical energy directly to the clothes or indirectly through wash liquid in the drum. The clothes mover can typically be moved in a reciprocating rotational movement. In some vertical axis washing machines, the drum rotates about a vertical axis generally perpendicular to a surface that supports the washing machine. However, the rotational axis need not be vertical. The drum can rotate about an axis inclined relative to the vertical axis.

As used herein, a "horizontal axis" washing machine refers to a washing machine having a rotatable drum, perforated or imperforate, that holds laundry items and washes the laundry items. In some horizontal axis washing machines, the drum rotates about a horizontal axis generally parallel to a surface that supports the washing machine. However, the rotational axis need not be horizontal. The drum can rotate about an axis inclined or declined relative to the horizontal axis. In horizontal axis washing machines, the clothes are lifted by the rotating drum and then fall in response to gravity to form a tumbling action. Mechanical energy is imparted to the clothes by the tumbling action formed by the repeated lifting and dropping of the clothes. Vertical axis and horizontal axis machines are best differentiated by the manner in which they impart mechanical energy to the fabric articles.

Regardless of the axis of rotation, a washing machine can be top-loading or front-loading. In a top-loading washing machine, laundry items are placed into the drum through an access opening in the top of a cabinet, while in a front-loading washing machine laundry items are placed into the drum through an access opening in the front of a cabinet. If a washing machine is a top-loading horizontal axis washing machine or a front-loading vertical axis washing machine, an additional access opening is located on the drum.

Aspects of the disclosure will be described in the context of a horizontal axis, front-load laundry treating appliance though this need not be the case. Aspects of the disclosure can have general applicability in laundry treating appliances with other configurations.

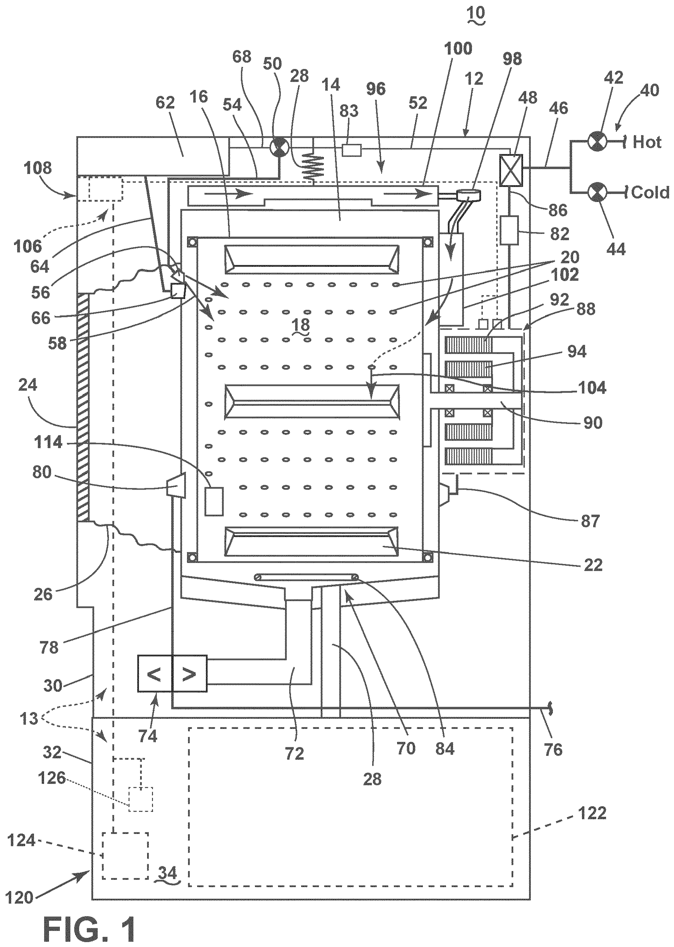

Referring now to FIG. 1, one exemplary laundry treating appliance 10 is shown in a schematic cross-sectional view. The laundry treating appliance 10 in the illustrated example is shown as a horizontal-axis combination washing and drying machine. It will be understood that aspects of the disclosure can be implemented in any appliance having any suitable configuration.

The laundry treating appliance 10 can include a structural support system. For example, the laundry treating appliance 10 includes a cabinet 12 defining an interior 13. The cabinet 12 can define a housing within which a laundry holding system resides. The cabinet 12 can include a chassis or a frame, to which decorative panels can be mounted, defining an interior enclosing component typically found in a conventional washing machine, such as motors, pumps, fluid lines, controls, sensors, transducers, and the like. Such components will not be described further herein except as necessary for a complete understanding of the present disclosure. In the example shown, the laundry holding system is positioned within the cabinet 12.

In the example shown, the cabinet 12 includes an upper cabinet 30 and a pedestal 32 though this need not be the case. The upper cabinet 30 can be supported by the pedestal 32. The pedestal 32 can include an interior compartment 34. The interior compartment 34 can be part of the interior 13 of the cabinet 12. The interior compartment 34 can be selectively accessed from outside of the laundry treating appliance 10. For example, the pedestal 32 can include a door, removable panel, sliding drawer, or the like, thereby providing access to the interior compartment 34. While illustrated within the pedestal 32, it will be understood that aspects of the disclosure can be implemented anywhere within the cabinet 12, including in laundry treating appliances without pedestals.

The laundry holding system includes a tub 14 dynamically suspended within the structural support system of the cabinet 12 by a suitable suspension system 28 and a drum 16 provided within the tub 14, the drum 16 defining at least a portion of a laundry treating chamber 18. The drum 16 is configured to receive a laundry load including articles for treatment. As used herein, an "article" for treatment will refer to any article for treatment by any suitable system of the laundry treating appliance, including, but not limited to, a hat, a scarf, a glove, a sweater, a blouse, a shirt, a pair of shorts, a dress, a sock, a pair of pants, a shoe, an undergarment, a jacket, a blanket, a sheet, or the like.

The drum 16 can include a plurality of perforations 20 such that liquid can flow between the tub 14 and the drum 16 through the perforations 20. It is also within the scope of the present disclosure for the laundry holding system to include only one receptacle with the receptacle defining the laundry treating chamber for receiving the load to be treated. At least one lifter 22 can extend from a wall of the drum 16 to lift the laundry load received in the treating chamber 18 while the drum 16 rotates.

The laundry holding system can further include a door 24 which can be movably mounted to the cabinet 12 to selectively close both the tub 14 and the drum 16. A bellows 26 can couple an open face of the tub 14 with the cabinet 12, with the door 24 sealing against the bellows 26 when the door 24 closes the tub 14. A door lock can also be provided for limiting access to the interior 13 during a cycle of operation. One or more door sensors can also be provided for detecting a position of the door 24, such as "door open" or "door closed" in non-limiting examples.

The laundry treating appliance 10 can further include a washing circuit which can include a liquid supply system for supplying water to the laundry treating appliance 10 for use in treating laundry during a cycle of operation. The liquid supply system can include a source of water, such as a household water supply 40, which can include separate valves 42 and 44 for controlling the flow of hot and cold water, respectively. Water can be supplied through an inlet conduit 46 directly to the tub 14 or the drum 16 by controlling first and second diverter mechanisms 48 and 50, respectively. The diverter mechanisms 48, 50 can be a diverter valve having two outlets such that the diverter mechanisms 48, 50 can selectively direct a flow of liquid to one or both of two flow paths. Water from the household water supply 40 can flow through the inlet conduit 46 to the first diverter mechanism 48 which can direct the flow of liquid to a supply conduit 52. The second diverter mechanism 50 on the supply conduit 52 can direct the flow of liquid to a tub outlet conduit 54 which can be provided with a spray nozzle 56 configured to spray the flow of liquid 58 into the tub 14. In this manner, water from the household water supply 40 can be supplied directly to the tub 14. While the valves 42, 44 and the conduit 46 are illustrated exteriorly of the cabinet 12, it will be understood that these components can be internal to the cabinet 12.

The laundry treating appliance 10 can also be provided with a dispensing system for dispensing treating chemistry to the treating chamber 18 for use in treating the load of laundry according to a cycle of operation. The dispensing system can include a treating chemistry dispenser 62 which can be a single dose dispenser, a bulk dispenser, or an integrated single dose and bulk dispenser and is fluidly coupled to the treating chamber 18. The treating chemistry dispenser 62 can be configured to dispense a treating chemistry directly to the tub 14 or mixed with water from the liquid supply system through a dispensing outlet conduit 64. The dispensing outlet conduit 64 can include a dispensing nozzle 66 configured to dispense the treating chemistry into the tub 14 in a desired pattern and under a desired amount of pressure. For example, the dispensing nozzle 66 can be configured to dispense a flow or stream of treating chemistry into the tub 14 by gravity, i.e. a non-pressurized stream. Water can be supplied to the treating chemistry dispenser 62 from the supply conduit 52 by directing the diverter mechanism 50 to direct the flow of water to a dispensing supply conduit 68.

The treating chemistry dispenser 62 can include multiple chambers or reservoirs for receiving doses of different treating chemistries. The treating chemistry dispenser 62 can be implemented as a dispensing drawer that is slidably received within the cabinet 12, or within a separate dispenser housing which can be provided in the cabinet 12. The treating chemistry dispenser 62 can be moveable between a fill position, where the treating chemistry dispenser 62 is exterior to the cabinet 12 and can be filled with treating chemistry, and a dispense position, where the treating chemistry dispenser 62 are interior of the cabinet 12.

Non-limiting examples of treating chemistries that can be dispensed by the dispensing system during a cycle of operation include one or more of the following: water, enzymes, fragrances, stiffness/sizing agents, wrinkle releasers/reducers, softeners, antistatic or electrostatic agents, stain repellants, water repellants, energy reduction/extraction aids, antibacterial agents, medicinal agents, vitamins, moisturizers, shrinkage inhibitors, and color fidelity agents, and combinations thereof.

The laundry treating appliance 10 can also include a recirculation and drain system for recirculating liquid within the laundry holding system and draining liquid from the laundry treating appliance 10. Liquid supplied to the tub 14 through the tub outlet conduit 54 and/or the dispensing supply conduit 68 typically enters a space between the tub 14 and the drum 16 and can flow by gravity to a sump 70 formed in part by a lower portion of the tub 14. The sump 70 can also be formed by a sump conduit 72 that can fluidly couple the lower portion of the tub 14 to a pump 74. The pump 74 can direct liquid to a drain conduit 76, which can drain the liquid from the laundry treating appliance 10, or to a recirculation conduit 78, which can terminate at a recirculation inlet 80. The recirculation inlet 80 can direct the liquid from the recirculation conduit 78 into the drum 16. The recirculation inlet 80 can introduce the liquid into the drum 16 in any suitable manner, such as by spraying, dripping, or providing a steady flow of liquid. In this manner, liquid provided to the tub 14, with or without treating chemistry can be recirculated into the treating chamber 18 for treating the load of laundry within.

The liquid supply and/or recirculation and drain system can be provided with a heating system which can include one or more devices for heating laundry and/or liquid supplied to the tub 14, such as a steam generator 82, an inline heater 83 and/or a sump heater 84. Liquid from the household water supply 40 can be provided to the steam generator 82 through the inlet conduit 46 by controlling the first diverter mechanism 48 to direct the flow of liquid to a steam supply conduit 86. Steam generated by the steam generator 82 can be supplied to the tub 14 through a steam outlet conduit 87. The steam generator 82 can be any suitable type of steam generator such as a flow through steam generator or a tank-type steam generator. Alternatively, the sump heater 84 can be used to generate steam in place of or in addition to the steam generator 82. In addition or alternatively to generating steam, the steam generator 82 and/or sump heater 84 can be used to heat the laundry and/or liquid within the tub 14 as part of a cycle of operation.

It is noted that the illustrated suspension system, liquid supply system, recirculation and drain system, and dispensing system are shown for exemplary purposes only and are not limited to the systems shown in the drawings and described above. For example, the liquid supply, dispensing, and recirculation and pump systems can differ from the configuration shown in FIG. 1, such as by inclusion of other valves, conduits, treating chemistry dispensers, sensors, such as water level sensors and temperature sensors, and the like, to control the flow of liquid through the laundry treating appliance 10 and for the introduction of more than one type of treating chemistry. For example, the liquid supply system can include a single valve for controlling the flow of water from the household water source. In another example, the recirculation and pump system can include two separate pumps for recirculation and draining, instead of the single pump as previously described.

The laundry treating appliance 10 also includes a drive system for rotating the drum 16 within the tub 14. The drive system can include a motor 88, which can be directly coupled with the drum 16 through a drive shaft 90 to rotate the drum 16 about a rotational axis during a cycle of operation. The motor 88 can be a brushless permanent magnet (BPM) motor having a stator 92 and a rotor 94. Alternately, the motor 88 can be coupled to the drum 16 through a belt and a drive shaft to rotate the drum 16, as is known in the art. Other motors, such as an induction motor or a permanent split capacitor (PSC) motor, can also be used. The motor 88 can rotate the drum 16 at various speeds in either rotational direction.

The motor 88 can rotate the drum 16 at various speeds in opposite rotational directions. In particular, the motor 88 can rotate the drum 16 at tumbling speeds wherein the fabric items in the drum 16 rotate with the drum 16 from a lowest location of the drum 16 towards a highest location of the drum 16, but fall back to the lowest location of the drum 16 before reaching the highest location of the drum 16. The rotation of the fabric items with the drum 16 can be facilitated by the at least one lifter 22. Typically, the force applied to the fabric items at the tumbling speeds is less than about 1G. Alternatively, the motor 88 can rotate the drum 16 at spin speeds wherein the fabric items rotate with the drum 16 without falling. The spin speeds can also be referred to as satellizing speeds or sticking speeds. Typically, the force applied to the fabric items at the spin speeds is greater than or about equal to 1G. As used herein, "tumbling" of the drum 16 refers to rotating the drum at a tumble speed, "spinning" the drum 16 refers to rotating the drum 16 at a spin speed, and "rotating" of the drum 16 refers to rotating the drum 16 at any speed.

The laundry treating appliance 10 can optionally include a drying system 96. In such a case, the drying system 96 can be a closed loop or an open loop circuit. In the illustrated example, a closed loop system is shown wherein the drying system 96 includes an air recirculation conduit that is fluidly coupled to and recirculates air 104 through the treating chamber 18. The air recirculation conduit can include any or all of a blower 98, a condenser 100, or a heating element 102. The condenser 100 can be provided with a condenser drain conduit (not shown) that fluidly couples the condenser 100 with the pump 74 and the drain conduit 76. Condensed liquid collected within the condenser 100 can flow through the condenser drain conduit to the pump 74, where it can be provided to the recirculation and drain system. In an exemplary aspect, the drying system 96 can be provided adjacent an upper portion of the tub 14, though it will be understood that the drying system 96 need not be provided adjacent an upper portion of the tub 14, and can be provided at any suitable location adjacent the tub 14. It is further contemplated that an open loop circuit is implemented wherein air is heated, passes through the drum 16, and is exhausted out of the laundry treating appliance 10, in which case a condenser 100 is not necessary.

The laundry treating appliance 10 can also include a control system for controlling the operation of the laundry treating appliance 10 to implement one or more cycles of operation. The control system can include a controller 106 located within the cabinet 12 and a user interface 108 that is operably coupled with the controller 106. The user interface 108 can include one or more knobs, dials, switches, displays, touch screens and the like for communicating with the user, such as to receive input and provide output. The user can enter different types of information including, without limitation, cycle selection and cycle parameters, such as cycle options.

The laundry treating appliance 10 can also include a sanitization system 120 for removing or reducing odors, bacteria, or other pathogens. In the example shown, the sanitization system 120 includes a sanitizing compartment 122 formed within the interior compartment 34 of the pedestal 32. It will be understood that aspects of the sanitizing compartment 122 are not limited to the pedestal 32. The sanitizing compartment 122 can be located anywhere within the interior 13 of the cabinet 12. While the sanitizing compartment 122 is illustrated as being smaller than the interior compartment 34 of the drawer 36, it will be understood that the sanitizing compartment 122 can have any suitable size, including the entire interior compartment 34.

The sanitization system 120 can include at least one detection sensor 124. The detection sensor 124 is illustrated within the sanitizing compartment 122 though this need not be the case. The detection sensor 124 can be positioned in any suitable location within or on the laundry treating appliance 10, including within the interior 13 of the cabinet 12 or on an exterior surface of the cabinet 12. Any number of detection sensors 124 can be provided. The detection sensor 124 can be in the form of a visual sensor, an acoustic sensor, an optical sensor, a proximity sensor, a door position sensor, a chemical sensor, or the like. The detection sensor 124 can be configured to sense or detect at least one of a presence of an article, an identification of an article, a size of an article, or a chemical property. As used herein, sensing or detecting a "chemical property" will refer to the sensing of a material type (e.g. plastic vs. glass), a chemical (e.g. bleach), a bacteria, a bacteria count or concentration, an organic compound, an organic compound concentration, a volatile organic compound (VOC), a VOC count or concentration, or the like, in non-limiting examples. As used herein, sensing or detecting an "identification of an article" will refer to the sensing of an item category (e.g. baskets, clothing, containers, compartment sidewall, etc.), an item type (e.g. shirt vs. shoes), an item size (e.g. "small item," "large item," "length: 18 inches," etc.), an item shape (e.g. rounded edges or sharp corners), or the like, in non-limiting examples.

In an example where the detection sensor 124 is provided within the sanitizing compartment 122, the detection sensor 124 can be configured to detect a presence of an article within the sanitizing compartment, an identification of an article within the sanitizing compartment, or a chemical compound within the sanitizing compartment, including a detection of at least one of airborne bacteria or volatile organic compounds within the sanitizing compartment.

The sanitization system 120 can also include an ionizing light source 126 (hereafter "light source 126"). The light source 126 can have any suitable form and be configured to emit ionizing radiation. As used here, "ionizing radiation" will refer to any frequency of electromagnetic radiation having sufficient energy for ionizing atoms or molecules encountered by that electromagnetic radiation as is understood in the art. Such ionizing radiation can include ultraviolet radiation or X-ray radiation in non-limiting examples. Such ionizing radiation can have a wavelength of 300 nm or smaller, or between 10 nm and 350 nm, or between 1 nm and 10 nm, in non-limiting examples.

It is contemplated that the light source 126 can be in the form of an ultraviolet light-emitting diode (UV LED) though this need not be the case. The light source 126 can have any suitable form. The light source 126 can also be a controllable light source having any or all of a variable frequency, a variable intensity, a variable time duration, a variable direction of emission, or the like. Any number of light sources 126 can be provided. Combinations of ionizing light sources 126 can also be utilized. In one non-limiting example, the light source 126 can include a first ionizing light source in the form of a UV LED emitting constant-frequency radiation and a second ionizing light source in the form of a UV LED emitting variable-frequency radiation. One or more ionizing light sources 126 can also be positioned anywhere within or on the laundry treating appliance 10.

The controller 106 can refer to the machine controller and any additional controllers provided for controlling any of the components of the laundry treating appliance 10, including the one or more sensors 124 and the ionizing light source 126. For example, the controller 106 can include the machine controller, a motor controller, or a sanitization system controller, or combinations thereof. Many known types of controllers can be used for the controller 106.In one example, the controller 106 can include a microprocessor-based controller that implements control software and sends/receives one or more electrical signals to/from each of the various working components to affect the control software. As an example, proportional control (P), proportional integral control (PI), and proportional derivative control (PD), or a combination thereof, a proportional integral derivative control (PID control), can be used to control the various components of the laundry treating appliance 10.

The sanitization system 120 is schematically illustrated as being in signal communication with the controller 106. The controller 106 is illustrated in the example of FIG. 1 as being located within the upper cabinet 30. Additionally or alternatively, the controller 106 can include a separate or dedicated controller for controlling portions of the sanitization system 120. Such a dedicated controller can be positioned anywhere within the cabinet 12, including within the drawer 36 in a non-limiting example.

The controller 106 can be coupled to the one or more detection sensors 124 provided in the sanitization system 120. The controller 106 can receive input from the one or more sensors 124. For example, the one or more detection sensors 124 can be configured to transmit a signal indicating at least one of a presence of an article within the sanitizing compartment, an identification of an article within the sanitizing compartment, or a chemical compound within the sanitizing compartment. The controller 106 in signal communication with the one or more sensors 124 can be configured to initiate, start, or commence a sanitization cycle within the sanitizing compartment 122 based at least on the transmitted signal from the sensor 124.

The controller 106 can further be coupled to the light source 126 provided in the sanitization system 120. The controller can select an operating parameter for at least the light source 126. The operating parameter can include at least one of an intensity of emitted radiation, a frequency of emitted radiation, a direction of emitted radiation, or a time duration for operating the light source 126, in non-limiting examples.

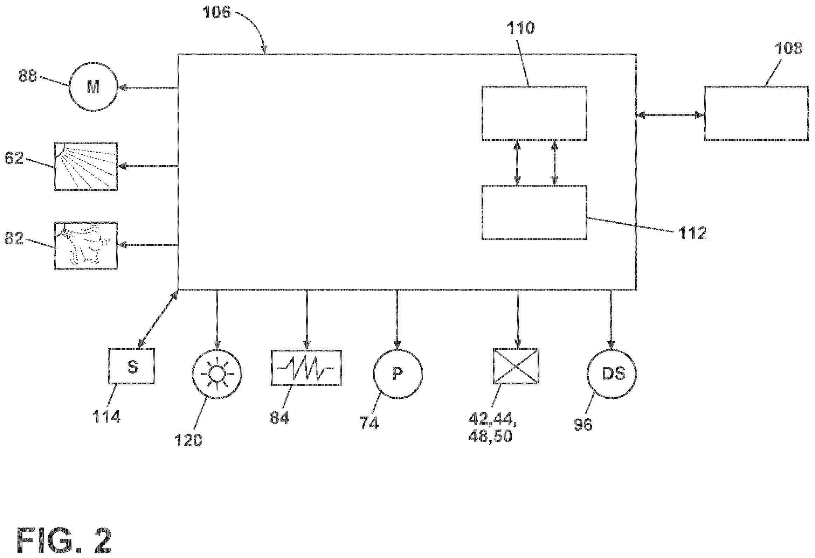

Turning to FIG. 2, the controller 106 can be provided with a memory 110 and a central processing unit (CPU) 112. The memory 110 can be used for storing the control software that is executed by the CPU 112 in completing a cycle of operation using the laundry treating appliance 10 and any additional software. Examples, without limitation, of cycles of operation include: wash, heavy duty wash, delicate wash, quick wash, pre-wash, refresh, rinse only, and timed wash. The memory 110 can also be used to store information, such as a database or table, and to store data received from one or more components of the laundry treating appliance 10 that can be communicably coupled with the controller 106. The database or table can be used to store the various operating parameters for the one or more cycles of operation, including factory default values for the operating parameters and any adjustments to them by the control system or by user input.

The controller 106 can be operably coupled with one or more components of the laundry treating appliance 10 for communicating with and controlling the operation of the component to complete a cycle of operation. For example, the controller 106 can be operably coupled with the motor 88, the pump 74, the treating chemistry dispenser 62, the steam generator 82, the sump heater 84, and the drying system 96 to control the operation of these and other components to implement one or more of the cycles of operation.

The controller 106 can also be coupled with one or more sensors 114 provided in one or more of the systems of the laundry treating appliance 10 to receive input from the sensors, which are known in the art and illustrated in FIG. 1 in a lower portion of the treating chamber 18 for exemplary purposes only. Non-limiting examples of sensors 114 that can be communicably coupled with the controller 106 include: a treating chamber temperature sensor, a moisture sensor, a weight sensor, a chemical sensor, a position sensor and a motor torque sensor, which can be used to determine a variety of system and laundry characteristics, such as laundry load inertia or mass. For example, the controller 106 can be coupled with one or more components of the sanitization system 120. For example, the controller 106 can be operably coupled to the one or more detection sensors 124 (FIG. 1) or the one or more ionizing light sources 126 (FIG. 1) as described above.

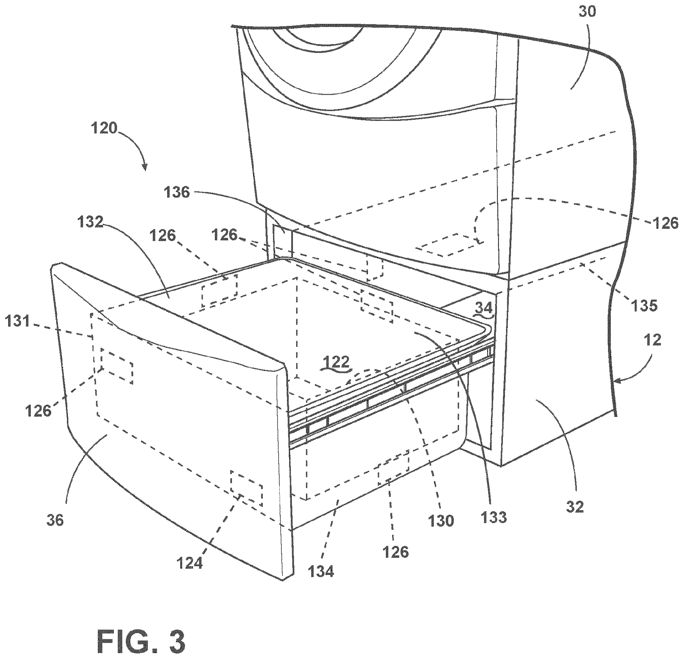

Referring now to FIG. 3, a perspective view of the laundry treating appliance 10 is shown with the pedestal 32 illustrated having a slidable drawer 36. It will be understood that aspects of the disclosure can apply to other pedestal configurations that do not include a drawer. The interior compartment 34 of the illustrated example is open to the exterior when the drawer 36 is slidably retracted or removed from the cabinet 12. It will be understood that the drawer 36 can also include a top cover, panel, or other closure providing selective access to the interior compartment 34 when the drawer 36 is slid away from the cabinet 12. In addition, the sanitizing compartment 122 of the illustrated example is located within the drawer 36 though this need not be the case.

An article 130 for treatment is schematically illustrated within the sanitizing compartment 122. The article 130 can include any suitable article, such as an article of clothing, a pair of shoes, or the like as described above. The article 130 can further include a non-clothing item such as a basket, a backpack, a treating chemistry container such as a detergent bottle, a cardboard box, or the like. One or more articles 130 can be positioned within the sanitizing compartment 122 as desired.

In the illustrated example, multiple ionizing light sources 126 as described above can be provided within the sanitizing compartment 122. More specifically, ionizing light sources 126 are positioned on each of a first wall 131, second wall 132, third wall 133, and fourth wall 134 of the drawer 36. At least one of the walls 131, 132, 133, 134 can define the sanitizing compartment 122. It is further contemplated that light sources 126 can be provided on interior surfaces of the pedestal 32 external to the drawer 36. In the example shown, light sources 126 are positioned on a top surface 135 and a side surface 136 within the pedestal 32.

Any or all of the walls 131, 132, 133, 134 or surfaces 135, 136 can have any suitable surface finish or property including transparent, translucent, opaque, mirror finish, or the like. In one example, the second wall 132 of the drawer confronting the light source 126 can be transparent to provide for emission of light from the light source 126 into the sanitizing compartment 122. In another example, all drawer walls 131, 132, 133, 134 can include a mirror finish to provide for increased exposure of the article 130 to ionizing radiation from the light sources 126. In this manner, one or more light sources can be coupled to at least one wall within the sanitizing compartment 122. The one or more light sources can emit ionizing radiation into the sanitizing compartment 122.

In the illustrated example, the detection sensor 124 is also provided within the sanitizing compartment 122 though this need not be the case. It will be understood that one or more detection sensors 124 and one or more light sources 126 can be provided in the laundry treating appliance 10. In addition, the one or more detection sensors 124 and/or the one or more light sources 126 can be in signal communication with the controller 106 (FIG. 1). Such connections are not shown in FIG. 3 merely for visual clarity.

With general reference to FIGS. 1-3, in one example of operation, a user can place the article 130 into the sanitizing compartment 122 and close the drawer 36. The controller 106 can determine a need for initiating a sanitization cycle of the laundry treating appliance 10 based at least on the signal from the detection sensor 124. For example, the detection sensor 124 can detect at least one of a presence of the article 130 (e.g. "sanitizing compartment empty" or "item present in sanitizing compartment"), an identification of the article 130 (e.g. "laundry basket" or "shoes, quantity 2"), a size of the article 130 (e.g. "large item,"), a property of the article 130 (e.g. "empty laundry basket" or "metal and plastic detected"), a weight of the article 130, or a chemical property within the sanitizing compartment 122 (e.g. "odor detected" or "volatile organic compound detected"), in non-limiting examples. The detection sensor 124 can transmit a signal to the controller 106 indicative of the detected presence, identification, property, or the like. The controller 106 can optionally be communicatively coupled to a database within or external to the laundry treating appliance 10 having a list of parameters for comparison with the transmitted signal from the detection sensor 124. The controller 106 can optionally be communicatively coupled with a user interface of the laundry treating appliance 10 and configured to receive user input regarding parameters of the article 130. In this manner, the controller 106 can be configured to determine a need for initiating a sanitizing cycle within the sanitizing compartment based at least on the signal from the detection sensor 124.

The controller 106 can also be configured to controllably operate the light source 126 within the laundry treating appliance 10 based on the determined need for initiating the sanitization cycle. For example, if the controller 106 has determined that a need exists for initiating the sanitization cycle, the controller 106 can select an operating parameter for at least the light source 126 such as an intensity of emitted radiation, a frequency of emitted radiation, a direction of emitted radiation, or a time duration for operating the light source 126. In non-limiting examples, the controller 106 can: transmit an operating signal to turn on one or more of the light sources 126; control a first group of light sources 126 to operate for a first time duration; control a second group of light sources 126 to operate for a second time duration; control multiple light sources 126 to illuminate in sequence; control multiple light sources 126 to illuminate simultaneously; control one or more of the light sources 126 to operate for a predetermined time duration; control one or more of the light sources 126 to continuously, smoothly, or step-wise increase an emitted frequency of UV radiation into the sanitizing compartment 122; or the like, or combinations thereof. In this manner, the controller can be configured to initiate an sanitization cycle within the sanitizing compartment 122 and operate the ionizing light source 126 based on the determined need for initiating the sanitization cycle.

The light sources 126 can illuminate the sanitizing compartment 122 and article 130 with ionizing radiation. Bacteria, chemical compounds, volatile organic compounds (VOCs), or the like can be deactivated or broken down by the ionizing radiation. In this manner, either or both of the sanitizing compartment 122 or the article 130 can be sanitized by direct illumination from the light sources 126 during a sanitization cycle initiated by the controller 106. In an example where a door lock is provided, the controller 106 can also operate the door lock to prevent opening of the sanitizing compartment 122 during a sanitizing cycle, including during operation of an ionizing light source.

It is further contemplated that the detection sensor 124 can transmit a signal indicating that a chemical compound level within the sanitizing compartment 122 has dropped below a predetermined threshold level. In one example, the detection sensor 124 can sense that a bacteria count has dropped below a predetermined threshold, or that a level of VOCs has dropped below a predetermined threshold. The controller 106 can receive the transmitted signal from the detection sensor 124 and determine that the sanitization cycle can be completed. The controller 106 can also controllably operate the light sources 126 to cease illumination. Optionally, a cycle, utilized by controller 106 to operate the light sources 126, can be that of a predetermined time period and strength based on a database of classified objects and relative need of sanitization.

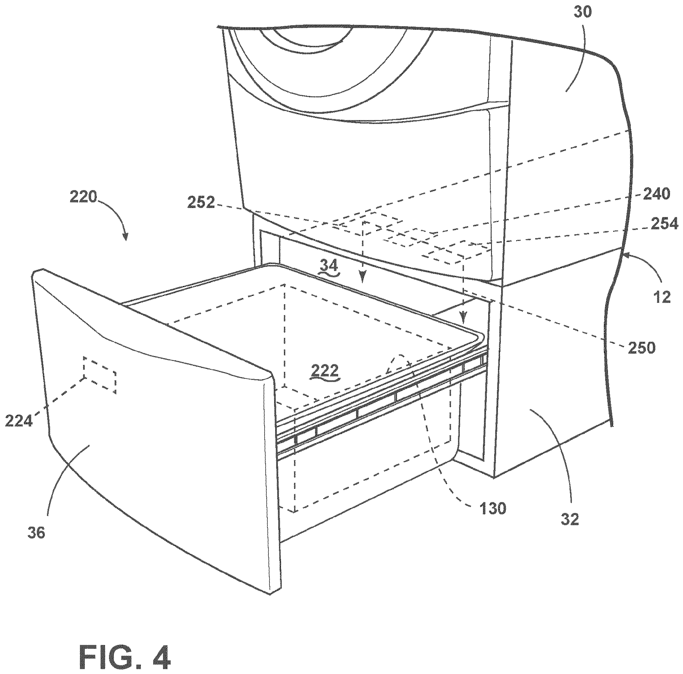

Turning to FIG. 4, the laundry treating appliance 10 is illustrated with another sanitization system 220 in accordance with various aspects described herein. The sanitization system 220 is similar to the sanitization system 120. Therefore, like parts will be identified with like numerals increased by 100, with it being understood that the description of the like parts of the sanitization system 120 applies to the sanitization system 220, except where noted.

The laundry treating appliance 10 includes the controller 106 (FIG. 1) and the sanitization system 220. The sanitization system 220 can include a sanitizing compartment 222, a detection sensor 224, and an ionizing light source 226 (or "light source" 226). An article 130 is illustrated within the sanitizing compartment 222. While the sanitizing compartment 222 is illustrated within the drawer 36 of the pedestal 32, it will be understood that the sanitizing compartment 222 can be positioned anywhere within or on the laundry treating appliance 10.

One difference compared to the sanitization system 120 is that the sanitization system 220 can include at least one photocatalytic component 240. A "photocatalytic component" as used herein will refer to any component configured to generate or emit oxidizing radicals when illuminated with electromagnetic radiation. Such oxidizing radicals can include, but are not limited to, ozone, hydroxyl, or triplet oxygen.

In one non-limiting example, the photocatalytic component 240 can generate oxidizing radicals when illuminated with ultraviolet light. In another non-limiting example, the photocatalytic component 240 can include titanium dioxide. In another non-limiting example, the photocatalytic component 240 can be in the form of a woven or nonwoven air filter including a photocatalytic material. In another non-limiting example, the photocatalytic component 240 can be in the form of a woven or nonwoven air filter having fibers coated with titanium dioxide. In still another non-limiting example, the photocatalytic component 240 can include a stack of air filters coated with photocatalytic material.

Another difference is that the sanitization system 220 can include a fluid flow path 250 that is fluidly coupled to the sanitizing compartment 222. In the example shown, the fluid flow path 250 can pass through the sanitizing compartment 222 between an inlet 252 and an outlet 254 though this need not be the case. Either or both of the inlet 252 or the outlet 254 can be fluidly coupled to ambient air outside the laundry treating appliance 10, or to air within the interior 13 of the laundry treating appliance 10, in non-limiting examples. In another non-limiting example, the inlet 252 and the outlet 254 can also be fluidly coupled to one another to form a recirculation circuit. In another non-limiting example, the fluid flow path 250 can include a conduit or passage external to the sanitizing compartment 222, wherein air is drawn out of the sanitizing compartment 222 via the outlet 254 and reintroduced back to the sanitizing compartment 222 via the inlet 252. In another non-limiting example, the fluid flow path 250 can be located within the sanitizing compartment 222 without use of an inlet or outlet. In another non-limiting example, the fluid flow path 250 can include a partition within the sanitizing compartment 222 through which air can circulate. It will be understood that the fluid flow path 250 can be provided anywhere within the laundry treating appliance 10.

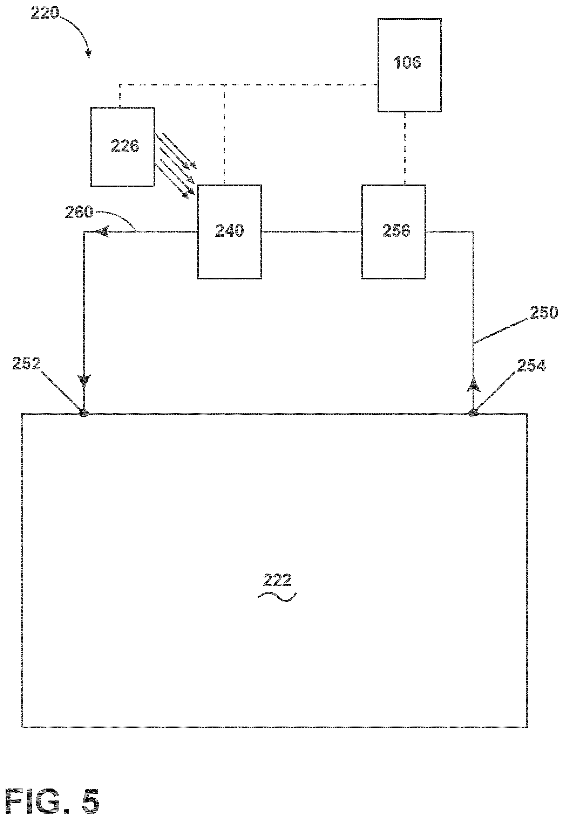

Turning to FIG. 5, the sanitization system 220 is shown in a schematic view. The photocatalytic component 240 can be positioned within the fluid flow path 250. The photocatalytic component 240 can also be positioned to confront the ionizing light source 226. Optionally, the light source 226 can also be positioned within the fluid flow path 250 though this need not be the case. Optionally, a fan 256 can be provided in the sanitization system 220 to draw air into the fluid flow path 250. The fan 256 can be positioned in any suitable location within the laundry treating appliance 10, including in the upper cabinet 30 or the pedestal 32.

With general reference to FIGS. 4-5, in one example of operation, the controller 106 can operate the fan 256 to draw air into the fluid flow path 250. The controller 106 can also operate the light source 226 to illuminate the photocatalytic component 240 with ionizing radiation (illustrated with arrows in FIG. 5). Oxidizing radicals can be generated by the illuminated photocatalytic component 240. The oxidizing radicals can mix into the fluid flow path 250 to define a treatment airflow 260. The treatment airflow 260 can be a mixture or enrichment of air, including ambient air, with the oxidizing radicals. The treatment airflow 260 can be directed along the fluid flow path 250 into the sanitizing compartment 222 by way of the inlet 252. The treatment airflow 260 can deactivate or break down bacteria, VOCs, or other chemical compounds within the sanitizing compartment 222. Optionally, the treatment airflow 260 can be drawn out of the sanitizing compartment 222 by way of the outlet 254. Optionally, the treatment airflow 260 can be recirculated by the fan 256 and back to the photocatalytic component 240 to have additional oxidizing radicals mixed into the treatment airflow 260.

In another example of operation, the controller 106 can also be configured to controllably operate at least one of the light source 226 or the fan 256 within the laundry treating appliance 10 based on the determined need for initiating the sanitization cycle. For example, if the controller 106 has determined that a need exists for initiating the sanitization cycle, the controller 106 can select an operating parameter for at least the light source 226 or the fan 256 such as, but not limited to, an intensity of emitted radiation, a frequency of emitted radiation, a direction of emitted radiation, a time duration for operating the light source 226, a speed of the fan 256, a direction of the fan 256, or a time duration for operating the fan 256. In non-limiting examples, the controller 106 can: transmit an operating signal to turn on the light source 226 or the fan 256; control the light source 226 or the fan 256 to operate for a predetermined time duration; control the light source 226 to continuously, smoothly, or step-wise increase or decrease an emitted frequency of UV radiation; control the fan 256 to continuously, smoothly, or step-wise increase or decrease a fan speed, or the like, or combinations thereof. In this manner, the controller can be configured to initiate an sanitization cycle within the sanitizing compartment 122 and operate at least one of the ionizing light source 126 or the fan 256 based on the determined need for initiating the sanitization cycle.

Aspects of the disclosure provide for a method of initiating a sanitization cycle in a laundry treating appliance 10 having a sanitizing compartment 122, 222. The method includes automatically sensing, via a sensor 124, 224, at least one of a presence of an article 130 within the sanitizing compartment 122, 222, an identification of the article 130 within the sanitizing compartment 122, 222, or a chemical property within the sanitizing compartment 122, 222. The method also includes receiving, in a controller 106, a signal from the sensor 124, 224 indicating the at least one of the presence of the article 130, the identification of the article 130, or the chemical property. The method also includes determining, in the controller 106, a need for initiating a sanitization cycle within the sanitizing compartment 122, 222 based on the signal from the sensor 124, 224. The method also includes controllably operating, by the controller 106, an ionizing light source 126, 226 within the laundry treating appliance 10 based on the determined need for initiating the sanitization cycle. Optionally, the method includes illuminating the article 130 within the sanitizing compartment 122, 222 with ultraviolet light from the ionizing light source 126, 226. Optionally, the method includes illuminating a photocatalytic component 240 with the ionizing light source 126, 226 to generate oxidizing radicals. Optionally, the controllably operating further includes operating a fan 256 to draw air into a fluid flow path 250 fluidly coupled to the sanitizing compartment 122, 222. Optionally, the controllably operating further comprises mixing the oxidizing radicals into the fluid flow path 250 to define a treatment airflow 260, and directing the treatment airflow 260 into the sanitizing compartment 122, 222. Optionally, the method includes selecting, by the controller 106, an operating parameter for the sanitization cycle after the determining the need for initiation. Optionally, the operating parameter includes one of an intensity of emitted radiation, a frequency of emitted radiation, a direction of emitted radiation, a time duration, or a fan speed. Optionally, the method includes automatically sensing, via the sensor 124, 224, a property of the article 130 within the sanitizing compartment 122, 222. Optionally, the method includes selecting, by the controller 106, an operating parameter for the sanitization cycle after the determining the need for initiation. Optionally, the selecting the operating parameter can be based on the signal from the sensor 124, 224.

Some additional operating examples will be described below with general reference to FIGS. 1-5. It will be understood that such operating examples are illustrative of aspects of the present disclosure and are not limiting in any way.

In one example, the detection sensor can include a camera or other visual detection system. If a user places a laundry basket within the sanitizing compartment, the camera can identify whether the laundry basket contains clothing items or is empty. If the laundry basket contains clothes, and if the controller determines a need for initiating a sanitization cycle, the controller can select operating parameters for at least one of the light source (e.g. frequency, intensity, or time duration) or the fan (e.g. fan speed, fan direction, or time duration) in such a way that accounts for the presence of potentially-delicate fabric items. If the controller determines a need for initiating a sanitization cycle and also determines that the clothing is not suitable for treatment in the sanitization system, the controller can determine not to initiate a sanitization cycle and provide a message for a user, such as "Remove Clothing."

In another example, the detection sensor can include a chemical sensor configured to detect a presence or concentration of VOCs. If a user places an article in the sanitizing compartment and closes the door, the detection sensor can automatically detect a concentration of VOCs within the sanitizing compartment. If the detected concentration is above a predetermined threshold, the controller can determine a need for initiating a sanitization cycle. In an example where the sanitization system includes a fan and a photocatalytic component, e.g. an air filter with photocatalytic material, the controller can adjust the fan speed or light source intensity based on the concentration of detected VOCs within the sanitizing compartment. The detection sensor can also continuously detect a concentration of VOCs within the sanitizing compartment during the cycle. If the concentration drops below a predetermined threshold, the controller can controllably operate the light source or fan to reduce intensity, reduce speed, or cease operation, in non-limiting examples.

In still another example, the detection sensors can include a proximity sensor, a camera, and a chemical sensor. If a user opens and closes the sanitizing compartment, the proximity sensor can transmit a signal to the controller indicative of whether an article has been placed within the sanitizing compartment. If an article has been placed within the sanitizing compartment, the camera can transmit a signal to the controller indicative of the item category (e.g. "pair of shoes, small, rounded edges, fabric exterior, metal present on exterior"). The chemical sensor can transmit a signal to the controller indicative of a presence of VOCs within the sanitizing compartment. The controller can determine a need for initiating a sanitization cycle based at least on the signals received from the proximity sensor, the camera, and the chemical sensor. The controller can also select operating parameters for at least the ionizing light source based on the received signals from the proximity sensor, camera, and chemical sensor.

The sanitization system can be utilized for sanitizing an empty sanitizing compartment. In one example, the detection sensors can include a proximity sensor, a chemical sensor, and a door sensor. If a user opens and closes the sanitizing compartment without placing an article inside, the proximity sensor can transmit a signal to the controller indicative that the compartment is empty. The chemical sensor can transmit a signal to the controller indicative of a presence of odors, bacteria, VOCs, or the like inside the sanitizing compartment. The door sensor can transmit a signal to the controller indicative of the sanitizing compartment being opened or closed, such as by sliding open or sliding closed the drawer of the pedestal. Additionally or alternatively, the laundry treating appliance can include a sanitizing compartment cleaning cycle. Such a cleaning cycle can be selected by a user in one example, or automatically determined for selection by the controller in another example. The controller can determine a need for initiating a sanitization cycle based on the signal from the proximity sensor, the signal from the chemical sensor, the signal from the door sensor, or from user selection of a sanitizing compartment cleaning cycle. In one example, the controller can select operating parameters for at least the ionizing light source based on the received signals or from the user selection of the sanitizing compartment cleaning cycle. In one example, the controller can select at least one of a low power, low intensity, or short time duration as operating parameters for the ionizing light source based on the signal from the proximity sensor indicative of the empty compartment.

Aspects of the disclosure provide for a variety of benefits. In laundry treating appliances with a pedestal configuration, the space within the pedestal can be utilized for sanitizing items such as laundry baskets, shoes, detergent bottles, or the like. Direct illumination of the sanitizing compartment with ultraviolet light can provide for ease of sanitizing for a variety of articles. The introduction of a treatment airflow into the sanitizing compartment can provide for a thorough sanitization of all surfaces exposed to the airflow that may not be directly illuminated by the light source.

Furthermore, the controller-determined initiation of a sanitization cycle provides for ease of operation and tailored sanitizing of articles. Compared to known systems that require user selection of a particular sanitizing cycle, or manual entry of parameters relating to the article to be sanitized, the sanitization system as described herein can use automatic sensor detection for sensing an article type, chemical compound, etc. and transmit a signal to the controller indicative of a need for sanitization. Such detection can be performed without need of additional user entry of parameters that may or may not accurately represent an actual need for sanitization.

To the extent not already described, the different features and structures of the various aspects can be used in combination with each other as desired, or can be used separately. That one feature can not be illustrated in all of the aspects is not meant to be construed that it cannot be, but is done for brevity of description. Thus, the various features of the different aspects can be mixed and matched as desired to form new aspects, whether or not the new aspects are expressly described.

While the present disclosure has been specifically described in connection with certain specific aspects thereof, it is to be understood that this is by way of illustration and not of limitation. Reasonable variation and modification are possible within the scope of the forgoing disclosure and drawings without departing from the spirit of the present disclosure. Hence, specific dimensions and other physical characteristics relating to the aspects disclosed herein are not to be considered as limiting, unless expressly stated otherwise.

Further aspects of the disclosure are provided by the subject matter of the following clauses:
1. A method of initiating a sanitization cycle in a laundry treating appliance having a sanitizing compartment, the method comprising automatically sensing, via a sensor, at least one of a presence of an article within the sanitizing compartment, an identification of the article within the sanitizing compartment, or a chemical property within the sanitizing compartment, receiving, in a controller, a signal from the sensor indicating the at least one of the presence of the article, the identification of the article, or the chemical property, determining, in the controller, a need for initiating a sanitization cycle within the sanitizing compartment based on the signal from the sensor, and controllably operating, by the controller, an ionizing light source within the laundry treating appliance based on the determined need for initiating the sanitization cycle.
2. The method of any preceding clause, further comprising illuminating the article within the sanitizing compartment with ultraviolet light from the ionizing light source.
3. The method of any preceding clause, further comprising illuminating a photocatalytic component with the ionizing light source to generate oxidizing radicals.
4. The method of any preceding clause, wherein the controllably operating further comprises operating a fan to draw air into a fluid flow path fluidly coupled to the sanitizing compartment.
5. The method of any preceding clause, wherein the controllably operating further comprises mixing the oxidizing radicals into the fluid flow path to define a treatment airflow, and directing the treatment airflow into the sanitizing compartment.
6. The method of any preceding clause, further comprising selecting, by the controller, an operating parameter for the sanitization cycle after the determining the need for initiation.
7. The method of any preceding clause, wherein the operating parameter comprises one of an intensity of emitted radiation, a frequency of emitted radiation, a direction of emitted radiation, a time duration, or a fan speed.
8. The method of any preceding clause, further comprising automatically sensing, via the sensor, a property of the article within the sanitizing compartment.
9. The method of any preceding clause, further comprising selecting, by the controller, an operating parameter for the sanitization cycle after the determining the need for initiation.
10. The method of any preceding clause, wherein the selecting the operating parameter is based on the signal from the sensor.
11. The method of any preceding clause, wherein the controllably operating further comprises operating a first light source for a first time interval and operating a second light source for a second time interval, with the first time interval and the second time interval being one of simultaneous or sequential.
12. A laundry treating appliance comprising a cabinet defining an interior, a rotatable treating chamber located within the interior, at least one wall within the interior defining a sanitizing compartment, an ionizing light source within the interior, a sensor configured to transmit a signal indicating at least one of a presence of an article within the sanitizing compartment, an identification of an article within the sanitizing compartment, or a chemical compound within the sanitizing compartment, and a controller in signal communication with the ionizing light source and the sensor, with the controller configured to initiate a sanitization cycle within the sanitizing compartment and operate the ionizing light source based at least on the transmitted signal from the sensor.
13. The laundry treating appliance of any preceding clause, wherein the ionizing light source comprises an ultraviolet light source.
14. The laundry treating appliance of any preceding clause, wherein the sensor comprises one of a visual sensor or a proximity sensor, with the sensor configured to detect the presence of an article located within the sanitizing compartment.
15. The laundry treating appliance of any preceding clause, wherein the sensor comprises a chemical sensor configured to detect at least one of airborne bacteria or volatile organic compounds within the sanitizing compartment.
16. The laundry treating appliance of any preceding clause, further comprising a photocatalytic component confronting the ionizing light source and configured to emit oxidizing radicals.
17. The laundry treating appliance of any preceding clause, further comprising a fluid inlet and a fluid outlet each fluidly coupled to the sanitizing compartment, with a fluid flow path passing through the sanitizing compartment between the fluid inlet and the fluid outlet.
18. The laundry treating appliance of any preceding clause, further comprising a fan operably coupled to the controller and configured to draw in air along the fluid flow path, mix the oxidizing radicals into the fluid flow path to define a treatment airflow, and direct the treatment airflow into the sanitizing compartment along the fluid flow path.
19. The laundry treating appliance of any preceding clause, wherein the cabinet comprises a pedestal and an upper cabinet supported on the pedestal, with the sanitizing compartment located within the pedestal and the rotatable treating chamber located within the upper cabinet.
20. The laundry treating appliance of any preceding clause, wherein the controller is configured to select an operating parameter comprising one of an intensity of emitted radiation, a frequency of emitted radiation, a direction of emitted radiation, a time duration, or a fan speed.
21. The laundry treating appliance of any preceding clause, wherein the ionizing light source is coupled to the at least one wall within the sanitizing compartment.
22. The laundry treating appliance of any preceding clause, further comprising a second ionizing light source within the interior and in signal communication with the controller.
23. The laundry treating appliance of any preceding clause, wherein the controller is configured to controllably illuminate the ionizing light source and the second ionizing light source at least one of sequentially or simultaneously.

## Claims

1. A method of initiating a sanitization cycle in a laundry treating appliance (10) having a sanitizing compartment (122, 222), the method comprising:
sensing, via a sensor (124, 224), at least one of a presence of an article within the sanitizing compartment (122, 222), an identification of the article within the sanitizing compartment, or a property, preferably a chemical property, more preferably a chemical property of the article, within the sanitizing compartment;
receiving, in a controller (106), a signal from the sensor (124, 224) indicating the at least one of the presence of the article, the identification of the article, or the chemical property;
determining, in the controller (106), a need for initiating a sanitization cycle within the sanitizing compartment (122, 222) based on the signal from the sensor (124, 224); and
based on the determined need for initiating the sanitization cycle, controllably operating, by the controller (106), an ionizing device within the laundry treating appliance (10).

2. The method of claim 1, wherein the ionizing device is a light source (126, 226), and wherein the controllably operating further comprises illuminating at least one of the sanitizing compartment (122, 222) or the article with ultraviolet light from the ionizing light source (126, 226).

3. The method of claim 2, further comprising illuminating a photocatalytic component (240) with the ionizing light source (126, 226) to generate oxidizing radicals.

4. The method of any of the claims from 1 to 3, wherein the controllably operating further comprises operating a fan (256) to draw air into a fluid flow path (250) fluidly coupled to the sanitizing compartment (122, 222).

5. The method of claim 4, wherein the controllably operating further comprises mixing the oxidizing radicals into the fluid flow path (250) to define a treatment airflow (260), and directing the treatment airflow (260) into the sanitizing compartment (122, 222).

6. The method according to any one of the preceding claims, further comprising selecting by the controller (106) an operating parameter for the sanitization cycle, after the determining the need for initiation.

7. The method of claim 6, wherein the operating parameter comprises at least one of an intensity of emitted radiation, a frequency of emitted radiation, a direction of emitted radiation, a time duration, or a fan speed.

8. The method of claim 6 or 7, wherein the selecting the operating parameter is based on the signal from the sensor (124, 224).

9. The method of claim 1, further comprising sensing, via the sensor (124, 224), a property of the article within the sanitizing compartment (122, 222), to further determine a stop of the sanitization cycle.
